(19) Europäisches Patentamt
European Patent Office
Office européen des brevets

(11) Numéro de publication: **0 007 843**
**A1**

(12) # DEMANDE DE BREVET EUROPEEN

(21) Numéro de dépôt: **79400473.9**

(22) Date de dépôt: **10.07.79**

(51) Int. Cl.[3]: **C 07 C 97/10**
**C 07 C 149/42**

(30) Priorité: **13.07.78 FR 7820941**

(43) Date de publication de la demande:
**06.02.80 Bulletin 80/3**

(84) Etats Contractants Désignés:
**AT BE CH DE FR GB IT LU NL SE**

(71) Demandeur: **SYNTHELABO**
**1, avenue de Villars**
**F-75341 Paris Cedex 07(FR)**

(72) Inventeur: **Najer, Henry**
**2 Avenue Emile Acollas**
**F-75007 Paris(FR)**

(74) Mandataire: **Thouret-Lemaitre, Elisabeth**
**Service Brevets - SYNTHELABO 58, rue de la Glacière**
**F-75621 Paris Cedex 13(FR)**

(54) **Dérivés de propiophénone, leur application en thérapeutique et leur préparation.**

(57) Composés répondant à la formule (I)

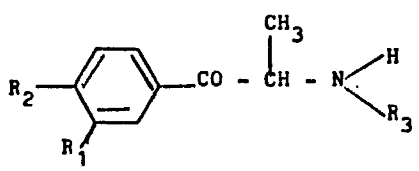

dans laquelle

$R_1$ représente un atome d'halogène, le radical trifluorométhyle ou trifluorométhyltio,

$R_2$ représente un atome d'hydrogène, un atome d'halogène le radical trifluorométhyle ou trifluorométhyltio et

$R_3$ représente un radical cyclobutyle ou tertiobutyle, à l'exception des composés pour lesquels $R_1$ = halogène, $R_2$ = H et $R_3$ = tertiobutyle. Ils sont préparés par réaction d'une amine de formule: $R_3NH_2$ avec un bromo-2-propiophénone.

Application en thérapeutique.

EP 0 007 843 A1

Croydon Printing Company Ltd.

DERIVES DE PROPIOPHENONE , LEUR APPLICATION EN THERAPEUTIQUE ET LEUR PREPARATION

La présente invention concerne des dérivés de propiophénone, leurs sels d'addition aux acides pharmaceutiquement acceptables, leur préparation et leur application en thérapeutique.

Les composés de l'invention répondent à la formule

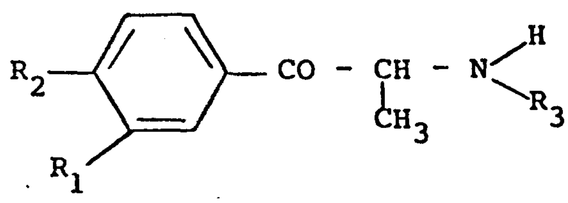

(I)

dans laquelle

$R_1$ représente un atome d'halogène, le radical trifluorométhyle ou trifluorométhylthio,

$R_2$ représente un atome d'hydrogène, un atome d'halogène, le radical trifluorométhyle ou trifluorométhylthio et

$R_3$ représente un radical cyclobutyle ou tertiobutyle, à l'exception des composés pour lesquels $R_1$= halogène, $R_2$ = H et $R_3$ = tertiobutyle.

Les composés de l'invention forment avec les acides pharmaceutiquement acceptables des sels qui font partie de l'invention.

On peut préparer les composés par réaction entre un composé de formule

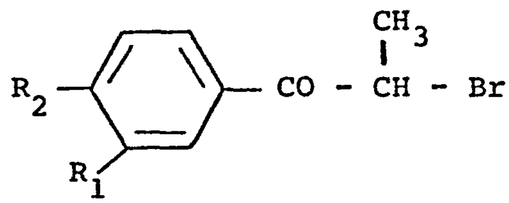

(II)

et un composé $R_3NH_2$ ; dans un solvant tel que l'acétonitrile à une température de 0 à 25°C.

Les composés (II) sont préparés à partir des nitriles

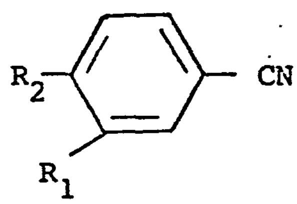

décrits par la Demanderesse dans son brevet 76 39033, selon le schéma réactionnel suivant

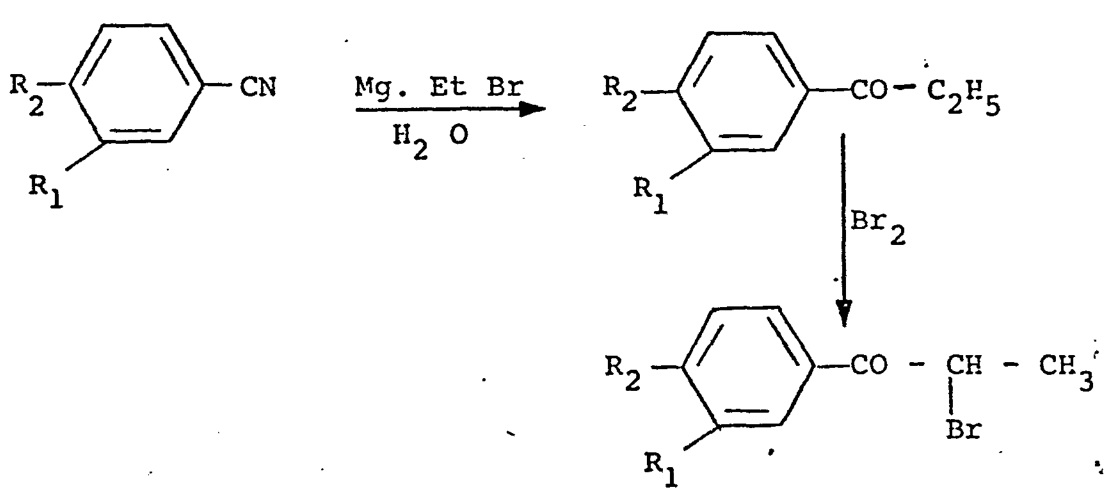


Les exemples suivants illustrent l'invention.

Les analyses et les spectres IR et RMN ont confirmé la structure des composés.

EXEMPLE 1 m-trifluorométhyl α -tertiobutylamino propiophénone et son chlorhydrate.

1. Trifluorométhyl-3' propiophénone.

Dans un erlenmeyer de 1000 ml avec chandelier à 2 branches, ampoule à brome de 100 ml, réfrigérant ascendant, (montage sec et protégé par des gardes à $CaCl_2$), on introduit 11 g (0,452 mole) de magnésium en tournures et 30 ml d'éther anhydre. On ajoute un cristal d'iode et introduit lentement une solution de 61,5 g (0,564 mole) de bromure d'éthyle. L'introduction terminée, on chauffe 2 heures à température de reflux puis on refroidit au bain d'eau glacée. On introduit alors, lentement et à froid, une solution de 63,7 g (0,372 mole) de métatrifluorométhylbenzonitrile.

Lorsque l'addition est terminée, on porte à la température de reflux pendant 4 heures. On refroidit et hydrolyse par 260 ml d'HCl 2N. On décante, lave la phase éthérée à l'eau, la sèche sur $MgSO_4$. On évapore à sec et distille le résidu sous vide.

Eb 0,03 = 60-70°C.

2. Chlorhydrate de trifluorométhyl-3' tertiobutylamino-2 propiophénone.

A une solution de 19,8 g (0,098 mole) de trifluorométhyl-3' propiophénone dans 70 ml de chlorure de méthylène, on introduit goutte à goutte une solution de 15,5g (0,097 mole) de brome dans 25 ml de chlorure de méthylène. On agite 1 heure 30 jusqu'à décoloration de milieu réactionnel. On évapore à sec à la température ambiante puis évapore 2 fois 50 ml de benzène. On ajoute alors 50 ml d'acétonitrile et filtre sur papier un léger insoluble. Le filtrat est alors ajouté goutte à goutte à une solution refroidie au bain d'eau glacée de 20,8 g de tertiobutylamine dans 60 ml d'acétonitrile. On agite ensuite 4 heures à 0°C et laisse reposer une nuit. On filtre le précipité formé,

le lave à l'éther et concentre à sec le filtrat. Le résidu est repris par de l'éther. On filtre l'insoluble et lave la solution éthérée à l'eau (2 fois). On sèche sur $MgSO_4$, filtre et évapore à sec. Le résidu est dissout dans 300 ml d'éther de pétrole bouillant et traité 15 mn avec du charbon végétal. On filtre le charbon sur papier et concentre à sec le filtrat.

Le résidu est dissous dans de l'acétone. On précipite le chlorhydrate par addition d'un excès d'éther chlorhydrique. On le filtre, l'essore, le lave abondamment à l'éther puis à l'acétone.

On le recristallise 2 fois dans de l'éthanol.

F ≐ 234°C (dec).

Dans le tableau I suivant sont représentés d'autres composés de l'invention préparés à titre d'exemples selon le même procédé.

TABLEAU I

| Composé n° | $R_1$ | $R_2$ | $R_3$ | Caractéristiques F (°C) du chlorhydrate |
|---|---|---|---|---|
| 1<br>77 233 | $CF_3$ | H | $C(CH_3)_3$ | 234 (déc) |
| 2<br>77 250 | Cl | H | 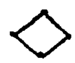 | 203-4 |
| 3<br>77 257 | $SCF_3$ | H | $C(CH_3)_3$ | 232 (déc) |
| 4<br>77 258 | $CF_3$ | H | 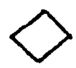 | 222-224 (déc) |
| 5<br>77 268 | $CF_3$ | Cl | $C(CH_3)_3$ | 255 (déc) |
| 6<br>77 304 | $CF_3$ | F | $C(CH_3)_3$ | 248 (déc) |
| 7<br>77 304 | $SCF_3$ | H | 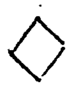 | 207-209 |
| 8<br>77 308 | $CF_3$ | F | 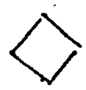 | 231-232 |

Les composés de l'invention ont été soumis à des essais pharmacologiques qui ont mis en évidence leurs propriétés psychotropes.

La toxicité des composés a été déterminée sur la souris, par voie intrapéritonéale. La DL 50 va d'environ 110 à environ 250 mg/kg/

Par ailleurs on a étudié l'action des drogues sur les pointes ponto-géniculo-occipitales lors du syndrôme réserpinique chez le rat curarisé, afin de mettre en évidence les propriétés antidépressives des composés.

Chez le chat, on enregistre une activité spécifique au niveau du pont, du noyau genouillé latéral et du cortex occipital qui fut appelée pointes ponto-géniculo-occipitales (P.G.O.).

Cette activité spontanée, (pointes P.G.O.), apparaissant lors du cycle veille sommeil, peut être induite par la réserpine, agent pharmacologique diminuant le taux des monoamines cérébrales.

Cette activité électroencéphalographique, modulée par de mécanismes neuronaux sous le contrôle de neurotransmetteurs synaptiques, constitue donc un test pharmacologique pour l'étude de l'action centrale des drogues.

Toutes les expériences sont réalisées de manière aiguë sur des chats des deux sexes de 2 à 3 kg.

Avant le début de la préparation chirurgicale effectuée sous narcose à l'éther, l'animal reçoit une injection intrapéritonéale de 0,75 mg/kg i.p. de réserpine.

Le chat est intubé pour permettre une ventilation artificielle.

Un anesthésique local (xylocaïneà 2%) est injecté au niveau des points de pression et des incisions.

Des canulés sont placées au niveau de la veine fémorale, pour l'injection des produits et l'infusion d'un agent curarisant, la gallamine triethiodide (Flaxédil$^{R}$), et de l'artère fémorale pour l'enregistrement de la pression artérielle.

Des electrodes monopolaires sont vissées au niveau cortical et des électrodes bipolaires coaxiales sont placées stéréotaxiquement au niveau des noyaux genouillés latéraux.

L'animal curarisé et ventilé artificiellement est maintenu à température constante tout au long de l'expérience.

L'électroencéphalogramme, l'électrocardiogramme et la pression artérielle sont enregistrés à l'aide d'un polygraphe Grass modèle 79 D.

Des doses croissantes (0,1 ; 0,3 ; 1 ; 3 ; 10 et 30 mg/kg) des produits à étudier sont injectées par voie intraveineuse toutes les 30 minutes, 4h 30 après l'administration de la réserpine.

Le nombre des pointes P.G.O. est comptabilisé automatiquement par périodes de 10 minutes et exprimé en pourcentage par périodes de 30 minutes en prenant la valeur obtenue lors des 30 minutes de contrôle comme référence (100%)

La dose efficace 50% des composés de l'invention varie de 1,5 à 3 mg/kg, le composé n° 2 (77 250) se révélant le plus actif.

Les résultats des essais précédents montrent que les composés de l'invention agissent sur le système nerveux

central et sont utiles pour le traitement des troubles de l'humeur et leurs symptômes associés.

Les composés de l'invention peuvent être présentés sous toute forme appropriée pour l'administration par voie orale, parentérale ou endorectale, par exemple sous forme de comprimés, de dragées, de gélules, de solutions buvables ou injectables, etc..., avec tout excipient approprié.

La posologie quotidienne peut aller de 10 à 100 mg.

Revendications

1. Composés répondant à la formule (I)

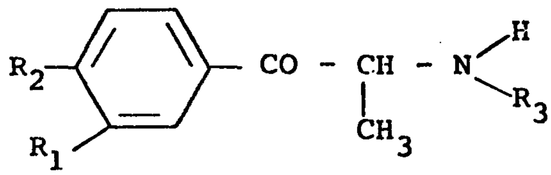

dans laquelle

$R_1$ représente un atome d'halogène, le radical trifluorométhyle ou trifluorométhyltio,

$R_2$ représente un atome d'hydrogène, un atome d'halogène, le radical trifluorométhyle ou trifluorométhyltio et

$R_3$ représente un radical cyclobutyle ou tertiobutyle, à l'exception des composés pour lesquels $R_1$ = halogène, $R_2$ = H et $R_3$ = tertiobutyle, ainsi que leurs sels d'addition aux acides pharmaceutiquement acceptables.

2. La m-chloro α-cyclobutylamino propiophénone et ses sels d'addition aux acides pharmaceutiquement acceptables.

3. Procédé de préparation des composés selon la revendication 1, procédé caractérisé en ce qu'on fait réagir un composé de formule (II)

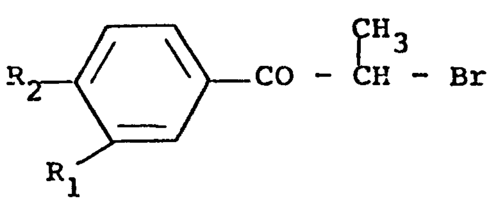

et un composé $R_3NH_2$.

4. Médicament caractérisé en ce qu'il contient un composé spécifié dans l'une quelconque des revendications 1 et 2.

Office européen des brevets

# RAPPORT DE RECHERCHE EUROPEENNE

Numéro de la demande

EP 79 40 0473

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR - A - 2 081 326 (THE WELLCOME FOUNDATION LTD.)<br>* Page 1, ligne 1 - page 2, ligne 9 *<br>-- | 1-4 | C 07 C 97/10<br>C 07 C 149/42 |
| A | FR - M - 3590 (RICHARDSON-MERRELL)<br>* Page 1, ligne 1 - page 2, ligne 1 *<br>---- | 1-4 | |

DOMAINES TECHNIQUES RECHERCHES (Int. Cl. 3)

C 07 C 97/10
C 07 C 149/42

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le present rapport de recherche a ete etabli pour toutes les revendications

| Lieu de la recherche | Date d'achèvement de la recherche | Examinateur |
|---|---|---|
| La Haye | 01-10-1979 | PAUWELS |